(19) Europäisches Patentamt
European Patent Office
Office européen des brevets

(11) Veröffentlichungsnummer: **0 186 815 A1**

# (12) EUROPÄISCHE PATENTANMELDUNG

(21) Anmeldenummer: **85115643.0**

(22) Anmeldetag: **09.12.85**

(51) Int. Cl.⁴: **C 07 C 43/13**, C 07 C 43/10, C 07 C 43/11, C 07 C 41/09, C 07 C 41/14

(30) Priorität: **20.12.84 DE 3446488**

(43) Veröffentlichungstag der Anmeldung: **09.07.86 Patentblatt 86/28**

(84) Benannte Vertragsstaaten: **BE CH DE FR GB IT LI NL**

(71) Anmelder: **HOECHST AKTIENGESELLSCHAFT, Postfach 80 03 20, D-6230 Frankfurt am Main 80 (DE)**

(72) Erfinder: **Scheffel, Günter, Dr., Holzfelderweg 14, D-8263 Burghausen (DE)**
Erfinder: **Obermeier, Reinhold, Töginger Strasse 65, D-8260 Mühldorf (DE)**

(54) **Verfahren zur Herstellung von Glykolalkylethern.**

(57) Beim neuen Verfahren werden Glykole mit Alkanolen und/oder Dialkylethern als Veretherungsmittel in Gegenwart von Lewis-Säuren als Katalysatoren umgesetzt und aus dem Umsetzungsprodukt, das im wesentlichen aus Glykolmonoalkylether und Glykoldialkylether, nicht-umgesetztem Glykol und nicht-umgesetztem Veretherungsmittel besteht, der Glykolmonoalkylether, der Glykoldialkylether oder eine Mischung der beiden Glykolether gewonnen. Bei diesem Verfahren werden relativ wenig unbrauchbare Nebenprodukte wie Dioxan gebildet.

EP 0 186 815 A1

## Verfahren zur Herstellung von Glykolalkylethern

Die Erfindung betrifft ein Verfahren zur Herstellung von Glykolalkylethern, wobei Glykolmonoalkylether, Glykoldialkylether oder Mischungen aus diesen beiden Ethern erhalten werden können.

Glykolalkylether, insbesondere jene der Ethylenglykole und Propylenglykole, sind aufgrund ihres hydrophilhydrophoben Charakters wertvolle Verbindungen. Sie werden beispielsweise als Lösemittel in der Lackindustrie, zur Herstellung von hydraulischen Flüssigkeiten, wie Bremsflüssigkeiten, Getriebeöle und dergleichen, und zur Herstellung von Reinigungsmitteln verschiedenster Art eingesetzt. Die Glykoldialkylether sind darüber hinaus als aprotische Lösemittel selektive Absorptions- und Extraktionsmittel.

Von den beiden Glykolethern sind die Monoalkylether leichter zugänglich. Sie werden allgemein durch Umsetzung von Alkanolen mit Alkylenoxiden, vorzugsweise Ethylenoxid und/oder Propylenoxid, in Gegenwart von alkalischen oder sauren Katalysatoren hergestellt, vergleiche beispielsweise deutsche Offenlegungsschrift 23 00 248.

Zur Herstellung der schwerer zugänglichen Glykoldialkylether sind im Lauf der Zeit eine Reihe von verschiedenen Verfahren entwickelt worden, die sich insbesondere hinsichtlich der eingesetzten Ausgangssubstanzen und des verwendeten Katalysators unterscheiden. Bei den schon

seit langem bekannten Verfahren, dem Williamson-Verfahren, sowie dem Dialkylsulfat-Verfahren sind bekanntlich Glykolmonoalkylether die Ausgangsverbindungen, die zur Herstellung der gewünschten Dialkylglykolether mit Alkylhalogeniden beziehungsweise Dialkylsulfaten umgesetzt werden. In beiden Fällen fallen äquimolare Mengen an Nebenprodukten an.

Bei dem in der deutschen Offenlegungsschrift 24 50 667 beschriebenen Verfahren zur Herstellung von Glykolethern werden Ethylenglykole und/oder Propylenglykole mit Olefinen unter Verwendung eines sauren Ionenaustauscherharzes als Katalysator umgesetzt. Nach der US-Patentschrift 4 146 736 werden Alkylenglykoldialkylether durch Umsetzung von Dialkylethern wie Dimethylether, mit Alkylenoxiden, vorzugsweise Ethylenoxid, in Gegenwart von Lewis-Säuren in homogener oder heterogener Phase hergestellt. Ein weiteres Verfahren zur Herstellung der in Rede stehenden Dialkylglykolether ist aus der US-Patentschrift 4 308 402 bekannt. Danach werden Oxaalkoxyalkanole an einem trägerfreien speziellen metallischen Nickelkatalysator zu den gewünschten Alkylenglykoldialkylethern umgesetzt.

Bei einem neueren, in der US-Patentschrift 4 321 413 beschriebenen Verfahren zur Herstellung von Ethylenglykoldialkylethern wird von Ethylenglykolmonoalkylethern und Dialkylethern als Veretherungsmittel ausgegangen, wobei die Veretherung in Gegenwart von sauren Ionenaustauscherharzen durchgeführt wird.

Die genannten Verfahren zur Herstellung der in Rede stehenden Glykolalkylether, insbesondere jene zur Herstellung der Diether, lassen noch mehrfach zu wünschen übrig.

Einige Verfahren liefern Glykolalkylether mit einer relativ großen Menge an unerwünschten Nebenprodukten und/ oder beruhen auf teuren Katalysatoren. Bei anderen wiederum ist es nicht möglich, nach Wunsch Glykolmonoalkylether, Glykoldialkylether oder Mischungen davon herzustellen.

Es besteht demnach die Aufgabe, ein Verfahren zur Herstellung von Glykolmonoalkylethern und Glykoldialkylethern vorzuschlagen, das auf billigen und leicht zugänglichen Ausgangsverbindungen und Katalysatoren beruht. Das Reaktionsprodukt soll relativ wenig unerwünschte Nebenprodukte, insbesondere wenig Dioxan, enthalten.

Das erfindungsgemäße Verfahren zur Herstellung von Glykolalkylethern ist dadurch gekennzeichnet, daß man ein Glykol mit einem Alkanol, einem Dialkylether oder mit einer Mischung aus den beiden als Veretherungsmittel in Gegenwart von Lewis-Säuren als Katalysatoren umsetzt, wobei als Glykol eine Verbindung der allgemeinen Formel

$$HO\!-\!\!-(CH_2CHR'O)_xH \qquad (I),$$

worin R' Methyl oder Wasserstoff und x eine Zahl von 1 bis 6 ist, als Alkanol eine Verbindung der allgemeinen Formel

$$ROH \qquad (II),$$

worin R eine Alkylgruppe mit 1 bis 18 Kohlenstoffatomen ist, und als Dialkylether eine Verbindung der allgemeinen Formel

$$R^1OR^2 \qquad (III),$$

worin $R^1$ und $R^2$ eine Alkylgruppe mit 1 bis 4 Kohlenstoffatomen ist, eingesetzt wird, und aus dem Umsetzungsprodukt, das im wesentlichen aus Glykolmonoalkylether und

Glykoldialkylether, nicht-umgesetztem Glykol und nicht-umgesetztem Veretherungsmittel besteht, den Glykolmonoalkylether, den Glykoldialkylether oder eine Mischung der beiden Glykolether gewinnt.

Als Glykole werden vorzugsweise solche eingesetzt, die sich nach Formel I ergeben, wenn x = 1 oder 2 ist und R' für Wasserstoff steht, das ist Ethylenglykol und Diethylenglykol.
Als Dialkylether werden vorzugsweise solche eingesetzt, die sich nach Formel III ergeben, wenn $R^1$ und $R^2$ eine Alkylgruppe mit 1 bis 3 Kohlenstoffatomen ist. $R^1$ und $R^2$ haben vorzugsweise die gleiche Bedeutung.
Als Alkanole werden vorzugsweise solche eingesetzt, die sich nach Formel II ergeben, wenn R eine Alkylgruppe mit 1 bis 3 Kohlenstoffatomen oder mit 12 bis 18 Kohlenstoffatomen ist. Die Alkanole, die diesen beiden Gruppen entsprechen, sind ebenso wie die erfindungsgemäß empfohlenen Glykole und Dialkylether besonders leicht erhältliche und billige Rohstoffe. Von den genannten Alkanolen sind jene mit 1 bis 3 Kohlenstoffatomen in der Alkylgruppe bevorzugt.
Von den erfindungsgemäß einzusetzenden Veretherungsmitteln sind die Alkanole und Mischungen aus den Alkanolen und Dialkylethern mit einer Alkanolmenge von mindestens 30 Gew.-%, vorzugsweise 40 bis 80 Gew.-%, bezogen auf die Mischung, bevorzugt. Im Falle solcher Mischungen soll $R = R^1 = R^2$ sein. Eine besonders geeignete Mischung besteht demnach aus Methanol und Dimethylether, Ethanol und Diethylether, Propanol und Dipropylether oder Isopropanol und Diisopropylether mit jeweils 40 bis 80 Gew.-% Alkanol und 20 bis 60 Gew.-% Dialkylether.

Die erfindungsgemäße Umsetzung der zu verethernden Glykole mit Alkanolen und Dialkylethern als Veretherungsmittel wird in Gegenwart von Lewis-Säuren vorgenommen, wobei eine heterogene oder homogene Katalyse verwendet wird. Die erfindungsgemäß einzusetzenden Lewis-Säuren können ihrer Zusammensetzung und ihrer Struktur nach sehr verschieden sein. Bevorzugt sind Lewis-Säuren in Form von Borhalogeniden, Phosphorhalogeniden und Phosphoroxyhalogeniden, in Form von Estern der Schwefelsäure, schwefeligen Säure, Phosphorsäure, phosporigen Säure und von Sulfonsäuren mit $C_1$- bis $C_3$-Alkanolen, in Form von Schwefelsäure, Flußsäure, Salzsäure, Fluoroborsäuren und Sulfonsäuren (das sind Protonensäuren, auch Wasserstoffsäuren oder Brönsted-Säuren genannt) und in Form von Alkalimetall-, Aluminium-, Eisen- oder Kupfersalzen dieser Säuren.

Als bevorzugte Vertreter der genannten Lewis-Säure-Gruppen seien genannt:
Aus der Gruppe der Borhalogenide, Phosphorhalogenide und Phosphoroxyhalogenide: $BF_3$, $PCl_3$ und $POCl_3$;
aus der Gruppe der Ester: $(CH_3)_2SO_4$ (Dimethylsulfat), $(CH_3)_2SO_3$ (Dimethylsulfit), $(CH_3)_3PO_4$ (Trimethylphosphat), $(CH_3)_3PO_3$ (Trimethylphosphit) und $CH_3FSO_3$ (Fluorsulfonsäuremethylester);
aus der Gruppe der Protonensäuren: $H_2SO_4$, HCl, $HBF_4$ (Tetrafluoroborsäure), $ClSO_3H$ (Chlorsulfonsäure), $FSO_3H$ (Fluorsulfonsäure) und $CF_3SO_3H$ (Trifluormethansulfonsäure);
aus der Gruppe der genannten Metallsalze: $Al_2(SO_4)_3$, $CuSO_4$, $Fe_2(SO_4)_3$, $KHSO_4$, $KAl(SO_4)_2$, $AlCl_3$, $FeCl_3$ und $(CF_3SO_3)_3Al$.
Die nachstehend angeführten Verbindungen sind besonders bevorzugte Katalysatoren: $PCl_3$, $POCl_3$, $(CH_3)_2SO_4$, $(CH_3)_2SO_3$, $CH_3FSO_3$, $H_2SO_4$, $HBF_4$, $ClSO_3H$, $FSO_3H$, $CF_3SO_3H$, $Al_2(SO_4)_3$, $Fe_2(SO_4)_3$, $KAl(SO_4)_2$ und $(CF_3SO_3)_3Al$.

Je nachdem, ob die verwendete Lewis-Säure im Reaktionsmedium gelöst ist oder nicht, liegt homogene beziehungs-

weise heterogene Katalyse vor. Von den oben aufgezählten Lewis-Säuren sind, wie ohne weiteres ersichtlich ist, die Metallsalze im Reaktionsmedium nicht löslich, während die Säuren, Nicht-Metallhalogenide und die Ester mehr oder weniger gut löslich sind. Die nicht-löslichen Katalysatoren können ebenso wie die löslichen, dem Reaktionsgut zugesetzt werden, also im Reaktionsgut selbst (in dispergierter Form) vorliegen, oder aufgebracht auf ein geeignetes Trägermaterial, beispielsweise Aluminiumoxid, Siliciumdioxid oder Kohle eingesetzt werden (Festbett-Katalyse). Von diesen Katalyse-Arten sind jene beiden bevorzugt, bei denen sich der Katalysator im Reaktionsgut (homogen oder dispergiert) befindet. Die homogene Katalyse ist besonders bevorzugt.

Bei der erfindungsgemäßen Umsetzung hängt die Reaktionsgeschwindigkeit im wesentlichen von der Katalysator-Konzentration und von der Reaktionstemperatur ab. Sie ist umso größer, je höher die Reaktionstemperatur und die Katalysator-Konzentration ist. Die wirksame Menge an Katalysator kann innerhalb eines weiten Bereiches variieren. Im Fall der homogenen Katalyse und ebenso im Fall der heterogenen Katalyse, bei welcher der Katalysator dispergiert vorliegt, beträgt die Katalysatormenge im allgemeinen 0,05 bis 5 Gew.-%, bezogen auf die eingesetzte Menge an zu veretherndér Verbindung. Werden weniger als 0,05 Gew.-% Katalysator eingesetzt, fällt die Reaktionsgeschwindigkeit unter den wirtschaftlich angestrebten Bereich, werden mehr als 5 Gew.-% eingesetzt, wird die Aufarbeitung des Reaktionsgemisches zunehmend unwirtschaftlicher. Die bevorzugte Katalysatormenge liegt bei 0,1 bis 3 Gew.-%, bezogen auf die eingesetzte Menge an zu verethernder Verbindung. Bei derjenigen heterogenen Katalyse, bei welcher der Katalysator als Festbett angeordnet ist und die Reaktionsmischung über das

Festbett geführt wird, wird Katalysator in einer solchen Menge eingesetzt, daß die Durchsatzmenge an Reaktionsmischung im Bereich von 0,5 bis 10 g, vorzugsweise 1 bis 5 g, pro Gramm Katalysator und pro Stunde liegt. Die Reaktionstemperatur des erfindungsgemäßen Verfahrens liegt im allgemeinen bei 150 bis 300 °C, vorzugsweise bei 180 bis 260 °C.

Die erfindungsgemäße Veretherungsreaktion wird auf einen Umsatz des eingesetzten Glykols von 20 bis 80 Gew.-%, vorzugsweise 30 bis 70 Gew.-%, gebracht. Es hat sich herausgestellt, daß es besonders vorteilhaft ist, diesen Umsatz nach einer Reaktionszeit von 10 bis 180 min, vorzugsweise 30 bis 120 min, zu erreichen und anschließend die Veretherungsreaktion abzubrechen. Die Beendigung der Umsetzung nach Erreichung des empfohlenen Umsatzgrades in der angegebenen Reaktionszeit kann einfach durch Abkühlen und/oder Neutralisieren des Umsetzungsproduktes (Zerstörung des Katalysators) herbeigeführt werden.

Die Einsatzmenge an zu verethernder Verbindung und Veretherungsmittel kann in relativ weiten Grenzen variieren. Im allgemeinen werden 0,5 bis 8 mol Veretherungsmittel, vorzugsweise 1 bis 5 mol Veretherungsmittel, pro mol Glykol eingesetzt. Die nachstehenden Reaktionsgleichungen mit Ethylenglykol (Monoethylenglykol), Methanol und Dimethylether als Reaktionskomponenten sollen die erfindungsgemäßen Veretherungsreaktionen näher veranschaulichen:

$$HOCH_2CH_2OH + CH_3OH \xrightarrow{\text{Katalysator}} CH_3OCH_2CH_2OH + H_2O$$

$$HOCH_2CH_2OH + 2CH_3OH \xrightarrow{\text{Katalysator}} CH_3OCH_2CH_2OCH_3 + 2H_2O$$

$$HOCH_2CH_2OH + CH_3OCH_3 \xrightarrow{\text{Katalysator}} CH_3OCH_2CH_2OH + CH_3OH$$

$$HOCH_2CH_2OH + CH_3OCH_3 \xrightarrow{\text{Katalysator}} CH_3OCH_2CH_2OCH_3 + H_2O$$

Das erfindungsgemäße Verfahren kann diskontinuierlich oder kontinuierlich durchgeführt werden, wobei sich je nach Reaktionskomponenten und Reaktionsbedingungen ein Dampfdruck einstellt, der im Bereich von 0,1 bis 7 MPa liegt.
Bei der diskontinuierlichen Reaktionsführung wird vorzugsweise so vorgegangen, daß die zu verethernde Verbindung, der Katalysator und das Veretherungsmittel vorgelegt werden, worauf erhitzt wird und bei Reaktionstemperatur bis zur Erreichung des Gleichgewichtszustandes oder bis zur Erreichung des gewünschten Umsetzungsgrades gehalten wird.
Die kontinuierliche Umsetzung wird vorzugsweise derart durchgeführt, daß ein Gemisch aus Katalysator, zu verethernder Verbindung und Veretherungsmittel innerhalb einer Wärmetauschzone aufgeheizt, zur Reaktion gebracht, nach Erreichen des gewünschten Umsatzes abgekühlt, gegebenenfalls von Katalysator befreit und destillativ oder extraktiv in gewünschte Fraktionen aufgetrennt wird. Nicht-umgesetzte Rohstoffanteile werden rückgeführt. Im Falle, daß Dialkylglykolether als bevorzugte Zielprodukte erzeugt werden sollen, werden auch Monoalkylglykolether rückgeführt. Der geringe Anteil an Nebenprodukten wird ausgeschleust.

Das erfindungsgemäße Verfahren beruht auf billigen und leicht zugänglichen Ausgangsverbindungen und Katalysatoren. Es läßt sich in einfacher Weise auf die Herstellung von Glykolmonoalkylethern und Glykoldialkylethern steuern. Es werden relativ wenig unbrauchbare Nebenprodukte wie Dioxan gebildet, was überraschenderweise insbesondere dann der Fall ist, wenn als Veretherungsmittel die angegebenen Alkanole oder die angegebenen Mischungen aus Alkanol und Dialkylether eingesetzt werden. Die verwendete saure Katalyse, insbesondere die homogene Katalyse, zeichnet sich durch eine hohe Wirkung aus. Bei der erfindungsgemäßen Umsetzung wird aufgrund von Gleichgewichtsreaktionen die wirtschaftlich höchstmögliche Ausbeute an Glykolalkylethern in relativ kurzer Zeit erreicht. Im Rahmen des erfindungsgemäßen Verfahrens werden vorzugsweise die Monoalkylether oder die Dialkylether von Monoethylenglykol und Diethylenglykol hergestellt.

Die Erfindung wird nun an Beispielen noch näher erläutert.

Beispiel 1

In einem mit Heizung und Rührer ausgerüsteten Autoklaven wurden vorgelegt:

5 mol (310 g) Monoethylenglykol
10 mol (320 g) Methanol, das sind 2 mol Veretherungsmittel pro mol Glykol
5 g Schwefelsäure, 98 gew.-%ig, das sind 1,0 Gew.-% Schwefelsäure, 100 gew.-%ig, bezogen auf das vorgelegte Glykol.

Es wurde mit Stickstoff gespült und der Autoklav verschlossen. Nun wurde die Mischung im Autoklaven unter Rühren auf 230 °C erhitzt, wobei sich ein Druck von 5 MPa einstellte. Bei der Reaktionstemperatur von 230 °C wurde eine Stunde lang unter Rühren gehalten und dann abgekühlt. Das Reaktionsprodukt wurde gaschromatographisch untersucht.
Der Glykolumsatz betrug 61,2 Gew.-%. Bezüglich Glykolmonoether, Glykoldiether, Dioxan und höhersiedenden Anteilen ergab die gaschromatographische Analyse das nachstehende Ergebnis in Gewichtsprozent (bezogen auf das Gewicht der Reaktionsmischung):

| | Gew.-% |
|---|---|
| Monoethylenglykolmonomethylether | 23,5 |
| Monoethylenglykoldimethylether | 9,9 |
| Dioxan | 0,8 |
| höhersiedende Anteile (Schwersieder) | 4,4. |

Beispiele 2 bis 19

Die Beispiele 2 bis 19 wurden analog dem Beispiel 1 durchgeführt.

Die eingesetzten Reaktionskomponenten, die Reaktionstemperatur und die Reaktionszeit sowie das gaschromatographische Ergebnis bezüglich Glykolumsatz und Glykolmonoalkylether (kurz Glykolmonoether genannt), Glykoldialkylether (kurz Glykoldiether genannt), Dioxan und Schwersieder im Reaktionsprodukt sind in der nachstehenden Tabelle zusammengefaßt; der Vollständigkeit halber enthält sie auch die entsprechenden Angaben zum Beispiel 1. In den Beispielen 10 bis 12 wurde als Veretherungsmittel jeweils eine Mischung aus Alkanol und Dialkylether eingesetzt, und zwar im Beispiel 10 43,0 Gew.-% Ethanol und 57,0 Gew.-% Diethylether, im Beispiel 11 78,0 Gew.-% Methanol und 22,0 Gew.-% Dimethylether und im Beispiel 12 41,0 Gew.-% Methanol und 59,0 Gew.-% Dimethylether.

In der Tabelle bedeuten:

RT = Reaktionstemperatur

RZ = Reaktionszeit

DME = Dimethylether ($CH_3OCH_3$)

DEE = Diethylether ($C_2H_5OC_2H_5$).

| Beispiel | Mol Veretherungsmittel pro Mol Glykol | Glykol | Katalysator (Gew.-%) | RT (°C) | RZ (min) | Glykolumsatz (Gew.-%) | Ergebnis in Gew.-% | | | |
|---|---|---|---|---|---|---|---|---|---|---|
| | | | | | | | Glykolmonoether | Glykoldiether | Dioxan | Schwersieder |
| 1 | 2 $CH_3OH$ | $HOCH_2CH_2OH$ | 1,0 $H_2SO_4$ | 230 | 60 | 61,2 | 23,5 | 9,9 | 0,8 | 4,4 |
| 2 | 2 $CH_3OH$ | $HOCH_2CH_2OH$ | 2,0 $HBF_4$ | 215 | 120 | 36,8 | 19,5 | 3,0 | - | - |
| 3 | 2 $CH_3OH$ | $HOCH_2CH_2OH$ | 1,0 $FSO_3H$ | 240 | 60 | 70,0 | 23,6 | 14,9 | 1,2 | 6,5 |
| 4 | 2 $CH_3OH$ | $HOCH_2CH_2OH$ | 1,0 $ClSO_3H$ | 230 | 60 | 43,0 | 22,8 | 3,5 | 0,2 | 0,2 |
| 5 | 2 $CH_3OH$ | $HOCH_2CH_2OH$ | 1,5 $Al_2(SO_4)_3$ | 230 | 60 | 43,5 | 20,5 | 6,5 | 0,1 | 0,2 |
| 6 | 1,5 $C_2H_5OH$ | $HOCH_2CH_2OH$ | 1,5 $Al_2(SO_4)_3$ | 240 | 120 | 55,3 | 27,9 | 8,8 | 1,0 | 5,4 |
| 7 | 1,8 Isopropanol | $HOCH_2CH_2OCH_2CH_2OH$ | 0,5 $Fe_2(SO_4)_3$ | 200 | 120 | 40,5 | 23,6 | 2,7 | 0,8 | - |
| 8 | 2 $CH_3OH$ | $HOCH_2CH_2OH$ | 5,0 $KAl(SO_4)_2$ | 230 | 60 | 38,9 | 18,5 | 5,8 | 0,3 | 0,4 |
| 9 | 2 $CH_3OH$ | $HOCH_2CH_2OH$ | 0,2 $(CH_3)_2SO_4$ | 260 | 30 | 57,8 | 28,5 | 7,8 | 0,5 | 0,2 |
| 10 | 1,1 $C_2H_5OH$ + 0,9 DEE | $HOCH_2CH_2OH$ | 2 $(CH_3)_2SO_3$ | 250 | 120 | 55,0 | 16,1 | 9,5 | 3,3 | 2,5 |
| 11 | 2,5 $CH_3OH$ + 0,5 DME | $HOCH_2CH_2OH$ | 1,5 $CF_3SO_3H$ | 220 | 90 | 51,5 | 19,3 | 4,0 | 0,1 | 1,3 |
| 12 | 1,25 $CH_3OH$ + 1,25 DME | $HOCH_2CH_2OH$ | 1,5 $CF_3SO_3H$ | 210 | 40 | 34,5 | 14,2 | 2,6 | - | - |
| 13 | 1,8 Isopropanol | $HOCH_2CH_2OCH_2CH_2OH$ | 0,5 $CH_3FSO_3$ | 200 | 120 | 70,0 | 29,5 | 10,1 | 5,8 | 1,5 |
| 14 | 1 Isopropanol | $HOCH_2CH_2OH$ | 0,5 $CH_3FSO_3$ | 180 | 120 | 38,4 | 28,4 | 4,8 | 0,2 | 0,6 |
| 15 | 2 $CH_3OH$ | $HOCH_2CH_2OH$ | 3 $PCl_3$ | 220 | 120 | 46,0 | 23,7 | 2,5 | 0,1 | 2,2 |
| 16 | 2 $CH_3OH$ | $HOCH_2CH_2OH$ | 3 $POCl_3$ | 220 | 120 | 35,8 | 18,6 | 2,1 | 0,1 | 1,5 |
| 17 | 2 $CH_3OH$ | $HOCH_2CH_2OH$ | 1,5 $CF_3SO_3H$ | 230 | 60 | 69,0 | 21,0 | 20,4 | 0,6 | 3,4 |
| 18 | 2 $CH_3OH$ | $HOCH_2CH_2OH$ | 1,5 $(CF_3SO_3)_3Al$ | 230 | 60 | 67,5 | 29,5 | 10,0 | 0,9 | 3,3 |
| 19 | 2 $CH_3OH$ | $HOCH_2CH_2OH$ | 0,05 $CF_3SO_3H$ | 250 | 90 | 60,5 | 26,0 | 7,9 | 1,0 | 4,2 |

Patentansprüche

1. Verfahren zur Herstellung von Glykolalkylethern, dadurch gekennzeichnet, daß man ein Glykol mit einem Alkanol, einem Dialkylether oder mit einer Mischung aus den beiden als Veretherungsmittel in Gegenwart von Lewis-Säuren als Katalysatoren umsetzt, wobei als Glykol eine Verbindung der allgemeinen Formel

$HO-(CH_2CHR'O)_xH$ (I),

worin R' Methyl oder Wasserstoff und x eine Zahl von 1 bis 6 ist, als Alkanol eine Verbindung der allgemeinen Formel ROH (II), worin R eine Alkylgruppe mit 1 bis 18 Kohlenstoffatomen ist, und als Dialkylether eine Verbindung der allgemeinen Formel $R^1OR^2$ (III), worin $R^1$ und $R^2$ eine Alkylgruppe mit 1 bis 4 Kohlenstoffatomen ist, eingesetzt wird, und aus dem Umsetzungsprodukt, das im wesentlichen aus Glykolmonoalkylether und Glykoldialkylether, nicht-umgesetztem Glykol und nicht-umgesetztem Veretherungsmittel besteht, den Glykolmonoalkylether, den Glykoldialkylether oder eine Mischung der beiden Glykolether gewinnt.

2. Verfahren nach Anspruch 1, dadurch gekennzeichnet, daß als Glykole Monoethylenglykol oder Diethylenglykol eingesetzt werden, als Alkanole Methanol, Ethanol, Propanol oder Isopropanol, und als Dialkylether Dimethylether, Diethylether, Dipropylether oder Diisopropylether.

3. Verfahren nach einem der Ansprüche 1 bis 2, dadurch gekennzeichnet, daß die Umsetzung bei einer Temperatur von 180 bis 260 °C durchgeführt wird.

4. Verfahren nach einem der Ansprüche 1 bis 3, dadurch gekennzeichnet, daß als Katalysator $PCl_3$, $POCl_3$, $(CH_3)_2SO_4$, $(CH_3)_2SO_3$, $CH_3FSO_3$, $H_2SO_4$, $HBF_4$, $ClSO_3H$, $FSO_3H$, $CF_3SO_3H$, $Al_2(SO_4)_3$, $Fe_2(SO_4)_3$, $KAl(SO_4)_2$ und $(CF_3SO_3)_3Al$ eingesetzt wird.

5. Verfahren nach einem der Ansprüche 1 bis 4, dadurch gekennzeichnet, daß Monoethylenglykol oder Diethylenglykol mit Methanol, Ethanol, Propanol oder Isopropanol oder mit einer Mischung aus Methanol und Dimethylether, Ethanol und Diethylether, Propanol und Dipropylether oder Isopropanol und Diisopropylether mit jeweils 40 bis 80 Gew.-% Alkanol und 20 bis 60 Gew.-% Dialkylether, unter Verwendung von 1 bis 5 mol Veretherungsmittel pro mol Glykol, bei einer Temperatur von 180 bis 260 °C in Gegenwart von 0,1 bis 3 Gew.-% $PCl_3$, $POCl_3$, $(CH_3)_2SO_4$, $(CH_3)_2SO_3$, $CH_3FSO_3$, $H_2SO_4$, $HBF_4$, $ClSO_3H$, $FSO_3H$, $CF_3SO_3H$, $Al_2(SO_4)_3$, $Fe_2(SO_4)_3$, $KAl(SO_4)_2$ und $(CF_3SO_3)_3Al$ als Katalysator umgesetzt wird, bis nach einer Reaktionszeit von 30 bis 120 min ein Umsatz des eingesetzten Glykols von 30 bis 70 Gew.-% erreicht ist.

0186815

Nummer der Anmeldung

Europäisches Patentamt

# EUROPÄISCHER RECHERCHENBERICHT

EP 85 11 5643

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | PATENTS ABSTRACTS OF JAPAN, Band 6, Nr. 208 (C-130) [1086], 20. Oktober 1982: & JP - A - 57 114 543 (MITSUI SEKIYU KAGAKU KOGYO K.K.) 16.07.1982 | 1 | C 07 C 43/13<br>C 07 C 43/10<br>C 07 C 43/11<br>C 07 C 41/09<br>C 07 C 41/14 |
| X | CHEMICAL ABSTRACTS, Band 87, Nr. 7, 15. August 1977, Seite 421, Nr. 52766v, Columbus, Ohio, US; & JP - A - 77 12 168 (MARUZEN OIL CO. LTD.) 05.04.1977; & CHEMICAL ABSTRACTS, Tenth Collective Index, Chemical Substances, Endorphin-Ethenaminium | 1 | |
| A | DE-B-2 640 505 (HOECHST)<br>* Anspruch; Beispiele 1,3 * | 1-4 | RECHERCHIERTE SACHGEBIETE (Int. Cl.4) |
| A | DE-C- 837 997 (HENKEL & CO.)<br>* Insgesamt * | 1-4 | C 07 C 41/00<br>C 07 C 43/00 |
| A,D | US-A-4 321 413 (S.H. VANDERPOOL)<br>* Zusammenfassung; Spalte 2, Zeilen 41-71 * | 1-4 | |
| A | US-A-2 122 129 (H.L. COX)<br>* Spalte 1, Zeilen 16-31 * | 1-4 | |

Der vorliegende Recherchenbericht wurde für alle Patentansprüche erstellt.

| Recherchenort | Abschlußdatum der Recherche | Prüfer |
|---|---|---|
| DEN HAAG | 25-03-1986 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument